(19) Europäisches Patentamt
European Patent Office
Office européen des brevets

(11) **EP 2 540 702 B1**

(12) **EUROPÄISCHE PATENTSCHRIFT**

(45) Veröffentlichungstag und Bekanntmachung des Hinweises auf die Patenterteilung:
**28.12.2016 Patentblatt 2016/52**

(51) Int Cl.:
*C07C 263/20* (2006.01)   *C07C 265/14* (2006.01)

(21) Anmeldenummer: **12170827.5**

(22) Anmeldetag: **05.06.2012**

(54) **Verfahren zur Gewinnung von Toluylendiisocyanat aus flüssigen Rückständen der Toluylendiisocyanatherstellung**

Method for the recovery of toluene diisocyanate from liquid residues of toluene diisocyanate production

Procédé de récuperation de toluylène-diisocyanate à partir de résidus liquides de fabrication de toluylène-diisocyanate

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.06.2011 DE 102011051467**

(43) Veröffentlichungstag der Anmeldung:
**02.01.2013 Patentblatt 2013/01**

(73) Patentinhaber: **Buss-SMS-Canzler GmbH
4133 Pratteln (CH)**

(72) Erfinder:
• **Hinz, Wolfgang
5400 Baden (CH)**

• **Gierecki, Lukas
79540 Lörrach (DE)**
• **Raouzeos, Georgios
4142 Münchenstein (CH)**

(74) Vertreter: **Griepenstroh, Jörg
Bockermann Ksoll
Griepenstroh Osterhoff
Patentanwälte
Bergstrasse 159
44791 Bochum (DE)**

(56) Entgegenhaltungen:
**DE-A1- 2 915 830     DE-C1- 4 430 951**

**Beschreibung**

Die vorliegende Anmeldung betrifft ein Verfahren zur kontinuierlichen thermischen Abtrennung und Gewinnung von Toluylen-2,4-diisocyanat (TDI) aus flüssigen Rückständen der TDI-Herstellung, durch Erhitzen der flüssigen Rückstände auf Verdampfungstemperatur und Eindampfen bis zum Feststoff ohne Zusatz von Hilfsmitteln, in einem durch Rühren mechanisch erzeugten Wirbelbett aus feinkörnigem, festem Gut.

TDI ist eines der wichtigsten Isocyanate ein wichtiges Zwischenprodukt der Kohlenstoffindustrie. Ende der 1930er Jahre erwies sich die Verbindung neben anderen Isocyanaten als eine ideale Ausgangsdistanz für Polyadditionsreaktionen und wurde von Otto Bayer und seinen Mitarbeitern als aussichtsreiches Produkt für die Herstellung von Schaumstoffen (Polyurethanen) eingestuft. Mittlerweile ist TDI in der chemischen Industrie ein wichtiges Zwischenprodukt für die Herstellung von Klebstoffen, Schaumstoffen für Matratzen und Polsterungen, Polyurethanen (z.B. für Schuhsohlen), Elastomeren, Beschichtungen und hochwertigen Lacken zur Verwendung in der Automobilindustrie, für Flugzeug- oder Triebwagenlackierungen.

Die Herstellung von TDI erfolgt durch die Phosgenierung von 2,4-Toluylendiamin in einem inerten Lösungsmittel, wie mono-Dichlorobenzol oder ortho-Dichlorobenzol. Nach der Reaktion werden zunächst das Lösungsmittel aus der Reaktionsmasse und anschließend das TDI abdestilliert. Bei der Trennung von TDI mittels Destillation bleibt als Sumpfprodukt ein noch etwas zähflüssiger Teer, der eine erhebliche Menge von TDI enthält (30 bis 70 Gew.-%, üblicherweise 50 ± 5 Gew.-%). Es ist höchst wünschenswert, das im Sumpf der Destillationskolonne verbleibende TDI zurückzugewinnen, weil es einen bedeutenden Teil der gesamten Ausbeute der Herstellung darstellt.

Die Problematik bei der Rückgewinnung von TDI aus dem Destillationssumpf ergibt sich insbesondere daraus, dass TDI zur Polymerisation und damit zur Bildung einer zähflüssigen Masse neigt, die Destillationsapparaturen bzw. Trockner verklebt und ein Rühren der zähflüssigen Masse erschwert.

Um das genannte Problem zu lösen wird in den Patentschriften DE 10 14 538, US 2,889,257, DE 11 33 721, DE 12 31 689, DE 12 43 178, US 3,729,386 und DE 43 17 669 der Einsatz von hochsiedenden Lösungsmitteln, Weichmachern oder Ölen, wie Bitumen, als Zugabe zum Destillationsrückstand vorgeschlagen, um die Abtrennung des darin befindlichen TDI durch Verdampfung zu ermöglichen. Dabei verhindern die hochsiedenden Lösungsmittel oder Weichmacher das Festwerden des Rückstandes im jeweiligen Verdampfungsgefäß, wodurch der Transport des Rückstandes aus dem Verdampfungsgefäß ermöglicht werden soll. Diese Verfahren sind jedoch aufwändig und teuer, weil die eingesetzten Zusatzmittel ihrerseits kostspielig gereinigt werden müssen, um wieder eingesetzt werden zu können. Außerdem belasten die meisten der eingesetzten Zusatzmittel die Umwelt und Gesundheit. Zudem gelingt es durch den Einsatz von hochsiedenden Zusatzmitteln oder Weichmachern häufig nicht vollständig, das Festwerden des Rückstands zu vermeiden.

Die US 3,457,291 beschreibt die Rückgewinnung von Isocyanaten in einem Drehrohrverdampfer, der mit Kugeln beladen ist. Die Kugeln sollen dazu dienen, dass der zähflüssige Rückstand gelockert und durchmischt wird, um die Entweichung des TDI daraus zu ermöglichen. Mit diesem Verfahren ist es jedoch nicht möglich, die TDI-Gewinnung kontinuierlich zu betreiben, da der mit Kugeln beladene Drehrohrverdampfer sich nur zum Chargenbetrieb eignet. Zudem weisen Drehrohrverdampfer eine nur relativ geringe Kapazität auf, so dass damit keine hohen Produktumsätze erzielt werden können. Der trockene Endrückstand muss zudem durch manuelle Handhabung entfernt werden. Nachteilig ist auch der offene Chargenbetrieb in einem Drehrohrverdampfer, der zu einer hohen Umweltbelastung führt.

Die DE 24 52 805 beschreibt ein Verfahren zur Abtrennung von Rückständen der TDI-Herstellung aus Gemischen mit TDI oder einem höher siedenden Lösungsmittel durch Eindampfen unter Rühren des Gemischs mit einem Wendelrührer. Auch hier sind die Nachteile des Verfahrens der quasikontinuierliche Betrieb, die kleine Kapazität pro installierte Volumeneinheit, die lange Verweilzeit im gerührten Verdampfer und die manuelle Handhabung des trockenen Endrückstands.

Weiterhin ist ein Verfahren bekannt (DE 29 15 830), bei dem die Gewinnung von TDI aus Destillationsrückständen der TDI-Herstellung in einem pneumatisch erzeugten Wirbelbett stattfindet. Das Verfahren ist jedoch apparativ aufwändig und auch energetisch ungünstig. Darüber hinaus ist die Ausbeute bezüglich der Rückgewinnung des Wertstoffs nur bis zu 96 Gew.-%. Nach der Rückgewinnung des Wertstoffs beträgt der Wertstoffrestgehalt im festen Rückstand mehr als 0,5 Gew.-%. Somit muss der feste Rückstand durch den vorhandenen Anteil an TDI als Sonderabfall klassifiziert werden.

Gemäß DE 44 30 951 wird der Destillationsrückstand aus der TDI-Herstellung mit bis zu 20 Gew.-% Bitumen bezogen auf das Gewicht des Destillationsrückstandes vermischt. Das Gemisch wird auf ein gerührtes Bett bestehend aus festem, körnigem Gut aufgebracht, wobei das Bett aus festem, körnigem Gut bereits auf die für die Verdampfung des im Destillationsrückstand beinhaltenden TDI erforderliche Temperatur aufgeheizt und gehalten wird. Es kommen sowohl Schnecken- als auch Schaufeltrockner zum Einsatz, die hierbei unter einem Druck von 10 bis 20 mbar betrieben werden. Dieser Druck erschwert jedoch das kontinuierliche Ein- und Ausschleusen des festen, körnigen Guts (Bettvorlage). Deshalb ist auch dieses Verfahren apparativ aufwändig und kostspielig. Der Einsatz des Rührbehälters als Rückstandstrockner stellt zwar ein einfaches Verfahren dar, jedoch ist das Verfahren für große Leistungen nicht geeignet und sein Betrieb ist durch den Einsatz von Bitumen aufwändig und kostspielig. Eine regelmäßige Inspektion der Belagsbildung im Inneren des Behälters und auf dem Rührer ist unabdingbar.

Das in der DE 43 17 669 beschriebene Verfahren sieht eine Vermischung des Destillationsrückstands aus der TDI-Herstellung mit bis zu 50 Gew.-% hochsiedenden Kohlenwasserstoffen (Bitumen), bezogen auf das Gewicht des Destillationsrückstands, sowie die Verarbeitung des Gemischs in einem kontinuierlich betriebenen, horizontal ausgerichteten, beheizten Kontakttrockner vor, der mit einer oder zwei ebenfalls beheizter langsam drehender Wellen ausgerüstet ist. Da gemäß dieser Schrift Kontakttrockner bevorzugt werden, die mit Einrichtungen zur Abreinigung der Welle und des Trocknergehäuses ausgestattet sind, können Ablagerungen des festen Rückstands an dem vom Gemisch berührten beheizten Oberflächen des Trockners nicht vermieden werden. Dadurch nimmt die Leistung des Trockners durch den höheren Widerstand bezüglich der Wärmeübertragung ab. Des Weiteren gestaltet der Gebrauch von Bitumen den Betrieb aufwändig und kostspielig.

Es war daher die Aufgabe der vorliegenden Erfindung ein Verfahren zur Abtrennung und Gewinnung von TDI aus flüssigen Rückständen der TDI-Herstellung bereitzustellen, das möglichst einfach handhabbar ist, bei dem das hergestellte TDI in wirtschaftlicher Weise quantitativ zurückgewonnen werden kann, das eine möglichst geringe Belastung für die Umwelt darstellt, bei dem eine hohe Verdampfungsleistung verwirklicht werden kann und bei dem auf dem Rührgerät bzw. auf der inneren Oberfläche einer Verdampfungsapparatur keine Belagsbildung durch rückständigen Teer stattfindet.

Die erfindungsgemäße Aufgabe wird gelöst durch ein Verfahren zur kontinuierlichen thermischen Abtrennung und Gewinnung von TDI aus flüssigen Rückständen der TDI-Herstellung in einem durch Rühren einer Schüttung aus feinkörnigem, festem Gut mit einem Rührorgan mechanisch erzeugten Wirbelbett aus dem feinkörnigen festen Gut in einem Wirbelbettbehälter, dadurch gekennzeichnet, dass

(a) die Umfangsgeschwindigkeit an der Spitze des Rührorgans stets eine Froude-Zahl von größer als 1 ergibt;

(b) die im Wirbelbettbehälter vorgelegte Schüttung des feinkörnigen, festen Gutes mindestens 20 bis maximal 80 Vol.-% des verfügbaren Volumens des Wirbelbettbehälters belegt;

(c) das Erhitzen der flüssigen Rückstände auf Verdampfungstemperatur durch direkten Kontakt mit dem vorgeheizten feinkörnigen, festen Gut der im Behälter vorgelegten und gewirbelten Schüttung erfolgt, wobei der Wärmeübertragungskoeffizient mindestens 120 W/m$^2$K beträgt; und

(d) das Rückmischverhältnis der Masse des Zulaufstroms der flüssigen Rückstände zur Masse der im Wirbelbettbehälter befindenden Schüttung mindestens 0,2 ist.

Unter dem Ausdruck "flüssiger Rückstand der TDI-Herstellung" versteht man den Destillationsrückstand bei der Destillation von TDI aus dem Reaktionsgemisch, das bei der Phosgenierung von 2,4-Toluylendiamin in einem inerten Lösungsmittel entsteht.

Bei dem erfindungsgemäßen Verfahren fällt ein fester Rückstand an, der sich vorzugsweise in einem feinkörnigen, festen Zustand befindet. Das feinkörnige feste Gut, das vor dem Verfahren in Form einer Schüttung in den Wirbelbettbehälter eingebracht wird, ist vorzugsweise ein Gut aus rieselfähigen Teilchen. Als feinkörniges festes Gut wird vorzugsweise der feinkörnige feste Rückstand eingesetzt, der bei einer vorangegangenen Abtrennung in dem erfindungsgemäßen Verfahren als Rückstand gewonnen wurde. Es ist jedoch prinzipiell auch denkbar, dass das feinkörnige feste Gut aus einem anderen Material als der feinkörnige, feste Rückstand besteht, das in seinen Eigenschaften diesem ähnelt. Das feinkörnige, feste Gut besteht vorzugsweise vor dem Einsatz im Verfahren aus Partikeln mit einem durchschnittlichen Durchmesser im Bereich von 0,1 bis 5 mm, stärker bevorzugt im Bereich von 0,5 bis 4 mm und am stärksten bevorzugt im Bereich von 0,5 bis 3 mm. Es ist besonders bevorzugt, dass die durchschnittliche Teilchengröße des feinkörnigen, festen Guts auch während des Verfahrens in dem genannten Bereich gehalten wird.

Unter einem mechanisch erzeugten Wirbelbett versteht man in der vorliegenden Erfindung, dass das feinkörnige feste Gut durch ein Rührorgan mechanisch verwirbelt wird, sodass die Feststoffpartikel des feinkörnigen, festen Guts mechanisch im Inneren des Wirbelbettbehälters in einen fluidisierten Zustand versetzt werden, die somit die Wirbelschicht bzw. das Wirbelbett bilden. Der Begriff "fluidisiert" weist in diesem Zusammenhang drauf hin, dass die (ehemalige) Schüttung nun Fluid-ähnliche Eigenschaften aufweist.

Unter der Umfangsgeschwindigkeit versteht man die Geschwindigkeit der fluidisierten Partikel des feinkörnigen, festen Guts an der Spitze des Rührorgans. Wie bereits erwähnt soll erfindungsgemäß die Umfangsgeschwindigkeit an der Spitze des Rührorgans stets eine Froude-Zahl von größer 1 ergeben. Besonders bevorzugt ist die Froude-Zahl jedoch nicht größer als 6, stärker bevorzugt nicht größer als 4 und am stärksten bevorzugt größer als 2. Die Froude-Zahl ist ein numerischer, dimensionsloser Index, welcher der Beschreibung des Bewegungsablaufs innerhalb eines gerührten Behälters dient. Er gibt das Verhältnis zwischen der Zentrifugalkraft und der Schwerkraft einer mechanisch gerührten Schüttung und wird durch die folgende Gleichung beschrieben:

$$\text{Froude-Zahl (Fr)} = \frac{r * \omega^2}{g},$$

wobei r der Radius des äußeren Mischwerkzeug, g die Erdbeschleunigung und ω die Winkelgeschwindigkeit darstellen. Die Froude-Zahl dient der Kennzeichnung kritischer Fließzustände. Eine Froude-Zahl von kleiner als 1 kennzeichnet beispielsweise ruhige Fließbedingungen, wobei die Schüttung eine reine Förderbewegung erfährt. Durch das Rührorgan wird die Schüttung bewegt, gedrückt, geschoben, gefördert und verdrängt. Die einzelnen Teilchen behindern sich. Die Platzwechselvorgänge sind beschränkt und der Freiheitsgrad der einzelnen Teilchen ist begrenzt. Eine Froude-Zahl von größer 1 gekennzeichnet ein Schleuder- und Wurf-Fließverhalten und ist erfindungsgemäß bevorzugt, da damit die Schüttung Wurf- und Wirbelbewegungen erfährt, die Platzwechselvorgänge zahlreich und stetig wechselnd sind und nichtzuletzt der Freiheitsgrad der Partikel der Schüttung erheblich gesteigert wird. Darüber hinaus gewährleistet eine ausreichende mechanische Verwirbelung die Aufrechterhaltung des Wirbelbetts, ohne dass die Partikel des feinkörnigen, festen Gutes durch Ablagerung von teerartigem Rückstand auf ihrer Oberfläche miteinander verkleben. Zudem gewährleistet eine Froude-Zahl von größer als 1, dass die Teilchen des feinkörnigen, festen Gutes, die durch die Ablagerung und Trocknung von teerartigem Rückstand auf der Oberfläche einer Massezunahme ausgesetzt sind, in dem erwünschten oben genannten Teilchendurchmesserbereich gehalten werden können. In anderen Worten gewährleistet eine Froude-Zahl von größer als 1 nicht nur eine ausreichende mechanische Verwirbelung sondern auch, dass die Teilchen durch das Rührorgan mechanisch zerkleinert werden. Die Froude-Zahl wird durch die geometrischen Gegebenheiten des Rührorgans und die Drehzahl bestimmt. Beide Größe sind bei einem geometrisch definierten Rührbehälter mit einem Rührorgan und bei definierten Betriebsbedingungen bezogen auf die Drehzahl des Rührorgans eindeutig definiert.

Unter dem Ausdruck "im Wirbelbettbehälter vorgelegte Schüttung des feinkörnigen, festen Guts mindestens 20 maximal 80 Vol.-% des verfügbaren Volumens des Wirbelbehälters belegt" versteht man, dass das Gesamtvolumen der Schüttung aus feinkörnigem, festem Gut vor Beginn des erfindungsgemäßen Verfahrens, nämlich nach dem Einbringen der Schüttung in den Wirbelbettbehälter, mindestens 20 % und maximal 80 % des verfügbaren Volumens des Wirbelbettbehälters einnimmt. Es ist weiterhin jedoch bevorzugt, dass die vorgelegte Schüttung des feinkörnigen, festen Gutes bis maximal 70 und stärker bevorzugt bis 60 Vol.-% des verfügbaren Volumens des Wirbelbettbehälters belegt. Ein zu geringer Volumenanteil des feinkörnigen, festen Gutes im Wirbelbettbehälter führt dazu, dass nur eine geringe Menge an flüssigem Rückstand pro Zeiteinheit in dem Wirbelbettbehälter eingesprüht werden kann, ohne dass es zu einer Belagsbildung auf dem Rührgerät bzw. der inneren Oberfläche des Wirbelbettbehälters durch teerartigen Rückstand kommt. Ein zu hoher Anteil des feinkörnigen, festen Gutes im Wirbelbettbehälter führt dazu, dass es zu keiner ausreichenden Verwirbelung des feinkörnigen, festen Gutes kommt, sodass keine ausreichende Durchmischung stattfinden kann.

Die flüssigen Rückstände müssen zur Verdampfung mindestens auf ihre Verdampfungstemperatur erhitzt werden. Das Erhitzen der flüssigen Rückstände auf Verdampfungstemperatur erfolgt vorzugsweise durch direkten Kontakt mit dem vorgeheizten feinkörnigen, festen Gut der im Behälter vorgelegten und gewirbelten Schüttung. Die Beheizung des feinkörnigen, festen Gutes erfolgt vorzugsweise durch Kontakt der verwirbelten Partikel mit der beheizten Innenwand des Wirbelbettbehälters. Es ist insbesondere bevorzugt, wenn die gesamte Innenwand des Wirbelbettbehälters beheizt wird. Trockene bzw. teilweise getrocknete Partikel des Wirbelbetts kommen in Kontakt mit der beheizten Innenwand des Gehäuses und werden so erhitzt. Vorzugsweise durch eine Materialzuführung eingeleiteter und vorzugsweise fein verteilter flüssiger Rückstand kommt in Kontakt mit den trockenen bzw. teilweise getrockneten Partikeln (feinkörniges, festes Gut) des Wirbelbetts. Die (teilweise) getrockneten Partikel bleiben dabei an den nassen Partikeln kleben. Da sich schnell viele trockene Partikel an die nassen Partikel anlagern können, wird eine Verteilung des flüssigen Anteils in den nassen Partikeln erzielt; mithin werden die nassen Partikel von den trockenen Partikeln umhüllt, wodurch verhindert wird, dass klebrige Phasen entstehen und dass das Nassgut in direkten Kontakt mit der Innenwand kommt, dort kleben bleibt und Krusten bildet.

Zudem wird den nassen Partikeln durch das Anlagern der trockenen und heißen Partikel Wärme zugeführt, sodass schnell eine Verdampfung des flüssigen Anteils einsetzen kann. Durch den Kontakt mit der beheizten Innenwand des Gehäuses, durch Kontakt mit erhitzten Partikeln des Wirbelbetts und durch Wärmeübertragung der im Innenraum vorhandenen Gase wird den zu trocknenden Partikeln weiter Wärme zugeführt, was zu weiterer Verdampfung des flüssigen Anteils in den zu trocknenden Partikeln führt. Die Verdampfung wird zudem durch die große freie zugängliche Oberfläche und durch die gute Durchmischung der Partikel im Wirbelbett begünstigt.

Da gemäß der vorliegenden Erfindung die Erzeugung des Wirbelbetts im Besonderen lediglich mechanisch erzeugt wird, kann der Trocknungsreaktor unter vermindertem Druck bzw. Vakuum gefahren werden. Dies erlaubt es, weniger Wärmeenergie für die Trocknung aufzuwenden, als dies etwa bei einem konventionellen Wirbelschichtreaktor der Fall ist.

Es ist weiterhin bevorzugt, dass der Wärmeübertragungskoeffizient mindestens 120 W/m²K und maximal 200 W/m²K beträgt. Das erfindungsgemäße Erhitzen und die erfindungsgemäßen Wärmeübertragungskoeffizienten von mindestens 120 W/m²K führen dazu, dass mit dem erfindungsgemäßen Verfahren eine äußerst hohe spezifische Verdampfungsleistung von mindestens 30 kg TDI pro Stunde und Quadratmeter erzielt werden kann. Unter dem Wärmeübertragungskoeffizient, auch Wärmeübergangskoeffizient oder Wärmeübergangszahl genannt, wird ein Proportionalitätsfaktor verstanden, der die Intensität des Wärmeübergangs an einer Grenzfläche bestimmt. Der Wärmeübertragungskoeffizient ist eine spezifische Kennzahl einer Konfiguration von Materialien bzw. von einem Material zu einer Umgebung in Form eines Fluids. Der Wärmeübertragungskoeffizient beschreibt hierbei die Fähigkeit eines Gases oder einer Flüssigkeit (hier die flüssigen Rückstände der Toluylendiisocyanatherstellung), Energie von der Oberfläche eines Stoffes (hier: das feinkörnige, feste Gut) abzuführen bzw. an die Oberfläche abzugeben. Der Wärmeübertragungskoeffizient hängt unter anderem von der spezifischen Wärmekapazität, der Dichte und dem Wärmeleitkoeffizienten des wärmeabführenden sowie des wärmeliefernden Mediums ab. Die Berechnung des Koeffizienten für die Wärmeleitung kann über den Temperaturunterschied der beteiligten Medien erfolgen. Der Wärmeübertragungskoeffizient kann durch die folgende Gleichung bestimmt werden, die, wenn sie nach α aufgelöst wird, den Wärmeübertragungskoeffizient α in der Dimension W/m². K angibt:

$$Q = \alpha \cdot A \cdot (T_1 - T_2) \cdot \Delta t,$$

wobei Q die übertragene Wärmemenge;
α der Wärmeübertragungskoeffizient;
A die betrachtete Kontaktfläche/benetzte Oberfläche;
$T_1$ und $T_2$ die Stofftemperaturen der beteiligten Medien und
Δt das betrachtete Zeitintervall ist.

Das Rückmischverhältnis der Masse des Zulaufstroms der flüssigen Rückstände zur Masse der im Wirbelbettbehälter befindenden Schüttung soll im Bereich von 0,2 bis 2 liegen, besonders bevorzugt im Bereich von maximal 0,5 bis 1,5 . Unter der Masse der im Wirbelbett befindenden Schüttung wird die mittlere Masse pro Zeiteinheit verstanden, die sich dann im zeitlichen Gleichgewicht befindet, wenn die Masse der Ablagerung der Rückstände auf dem festen Gut im zeitlichen Mittel gleich der Masse des entfernten festen Guts im zeitlichen Mittel ist.

Die Kombination des Merkmals des günstigen Verhältnisses der Masse der Zulaufmenge der flüssigen Rückstände zur Masse der im Wirbelbettbehälter befindenden Schüttung und des Merkmals eines Wärmeübertragungskoeffizienten von mindestens 120 W/m²K führt dazu, dass mit dem erfindungsgemäßen Verfahren die Bildung intermediärer viskoser, pastöser krusten- und schaumbildender Phasen vermieden werden kann. Auf diese Weise kommt es zu keiner Belagsbildung auf dem Rührgerät bzw. der inneren Oberfläche des Wirbelbettbehälters. Zudem müssen deshalb auch keine Zusatzmittel, wie hochsiedende Kohlenwasserstoffe oder Weichmacher zugesetzt werden. Deshalb ist das Verfahren wesentlich effizienter und belastet die Umwelt nicht in dem Maße wie die im Stand der Technik bekannten Verfahren.

Es ist erfindungsgemäß also auch bevorzugt, dass das erfindungsgemäße Verfahren ohne den Zusatz von Hilfsmitteln, wie hochsiedenden Kohlenwasserstoffen oder Weichmachern, wie beispielsweise Bitumen, durchgeführt wird.

Vorzugsweise wird das Wirbelbett im erfindungsgemäßen Verfahren bei Temperaturen von 150 bis 380°C, stärker bevorzugt von 170 bis 350°C und am stärksten bevorzugt von 205 bis 300°C gehalten. Eine Temperatur in diesem Bereich hat den Vorteil, dass immer ausreichend Verdampfungswärme vorhanden ist.

Vorzugsweise wird der Druck im Wirbelbettbehälter während dem erfindungsgemäßen Verfahren bei 25 mbar bis 1000 mbar, stärker bevorzugt 50 mbar bis 1000 mbar und am stärksten bevorzugt 100 mbar bis 1000 mbar gehalten. Der angegebene Druckbereich ist insbesondere vorteilhaft, da auch unter normalem Atmosphärendruck gearbeitet werden kann, sodass ein Zuführen flüssigen Rückstandes der TDI-Herstellung und ein Abführen von gebildeten feinkörnigem, festem Gut ohne aufwändiges Zwischenschalten von Ventilen oder Druckkammern zum Druckausgleich verwirklicht werden kann. Somit kann das Verfahren leichter kontinuierlich betrieben werden.

Die mittlere Verweilzeit der flüssigen Rückstände im Wirbelbett wird vorzugsweise von 30 Minuten bis 120 Minuten und stärker bevorzugt von 30 Minuten bis 180 Minuten gehalten.

Die entsprechenden angegebenen bevorzugten Temperaturen, Drücke und Verweilzeiten bringen den Vorteil mit sich, dass TDI aus den flüssigen Rückständen der TDI-Herstellung nahezu quantitativ entfernt werden kann. Somit enthält der gebildete feinkörnige, feste Rückstand (Gut) nahezu kein TDI mehr, sodass er nicht auf aufwändige Weise entsorgt werden muss.

Weiterhin ist bevorzugt, dass das mechanisch erzeugte Wirbelbett aus dem feinkörnigen, festen Gut durch ein an einer Rotorwelle angeordnetes Rotorblatt als spezielles Rührorgan erzeugt wird. Die Rotorwelle ist vorzugsweise im Innenraum eines Wirbelbettreaktors angebracht und dazu bestimmt, dass zu trocknende Material mittels an der Rotorwelle angeordneter Rotorblätter (oder "Rotorpaddel") in der Wirbelschichtzone aufzuwirbeln und von der Materialzuführung in Richtung zum Trockengutaustrag hin zu fördern. Das so gestaltete Rührorgan ist in der Lage, das von dem feinkörnigen festen Gut gebildete Wirbelbett vollständig zu durchmischen und somit permanent in einem körnigen, festen rieselfähigen Zustand zu halten.

Des Weiteren ist das erfindungsgemäße Verfahren vorzugsweise dadurch gekennzeichnet, dass das Wirbelbett ausschließlich aus feinkörnigem, festem Rückstand besteht.

Für die Zufuhr der flüssigen Rückstände der TDI-Herstellung in das Wirbelbett wird vorzugsweise eine Einstoffdüsebatterie verwendet und der Zulaufstrom der flüssigen Rückstände wird vorzugsweise mit einem Vordruck von grösser 1 bar auf das Wirbelbett eingedüst.

Zudem wird die Energie für die Beheizung der im Wirbelbettbehälter befindenden Schüttung des feinkörnigen, festen Gutes vorzugsweise ausschließlich über die beheizte Wand des Wirbelbettbehälters und über das beheizte spezielle Rührorgan in das Wirbelbett eingebracht.

Weiterhin ist es bevorzugt, dass das erfindungsgemäße Verfahren kontinuierlich in einem geheizten Schaufeltrockner mit Entgasungseinrichtung als Wirbelschichtbehälter durchgeführt wird. Der Schaufeltrockner verfügt vorzugsweise über einen Doppelmantel zur Beheizung und eine horizontale ebenfalls beheizte Welle. Vorzugsweise weist die Welle eine Geometrie auf, die gleichzeitig das im Schaufeltrockner befindende feinkörnige, feste Gut durch Rühren mechanisch verwirbelt und teilweise rückmischt. Am Gehäuse des Trockners befinden sich vorzugsweise Stutzen für den Zuführstrom des Destillationsrückstandes (der flüssige Rückstand der TDI-Herstellung) und ein oder mehrere weitere Stutzen für den Abzug des verdampften TDI und ein oder mehrere weitere Stutzen für den Abzug des trockenen festen Rückstandes. Vorzugsweise sind ebenso sonstige Stutzen für den Zu- und Ablauf des Heizmediums vorhanden.

Das erfindungsgemäße Verfahren wird vorzugsweise folgendermaßen durchgeführt: Im Prozessraum des Wirbelschichtbehälters wird feinkörniges, festes Gut vorgelegt und konditioniert. Die Konditionierung der vorgelegten Schüttung des feinkörnigen, festen Gutes umfasst das Vorheizen auf Betriebstemperatur, das Erzeugen des Wirbelbetts und das Anlegen des Betriebsdrucks. Der flüssige Rückstand aus der TDI-Herstellung wird vorzugsweise über mindestens zwei bis fünf Zulaufventile (Stutzen) in den konditionierten Prozessraum verteilt. Die Zulaufventile wirken vorzugsweise auch als Druckhalteventile, um eine Verdampfung von TDI in die Zulaufleitung mit gleichzeitiger Ausscheidung des festen Rückstandes und Verstopfung der Zulaufleitung zu vermeiden.

Die weitere Funktion der Zulaufventile ist das Aufsprühen des flüssigen Rückstandes auf das Wirbelbett aus dem feinkörnigen, festen Gut. Vorzugsweise findet beim Sprühen des Zulaufstroms in dem Prozessraum abhängig von der Temperatur und der Zusammensetzung des Zulaufstroms, eine spontane Flash-Verdampfung statt. Die Flash-Verdampfung tritt auf, wenn ein gesättigter Flüssigkeitsstrom unter einem bestimmten konstanten Druck gehalten wird und unter diesem Druck zumindest auf die Siedetemperatur der gesättigten Flüssigkeit aufgeheizt wird, dann wird der Flüssigkeitsstrom einer Druckreduzierung unterworfen, indem der Flüssigkeitsstrom durch ein Drosselventil oder eine andere Drosseleinrichtung durchfliesst. Wenn das Drosselventil oder die Drosseleinrichtung sich am Gehäuse eines Druckbehälter befindet (erfindungsgemäss dieser Druckbehälter ist der Trockner), tritt der Flash-Verdampfung innerhalb des Prozessraums des Trockners auf. Wenn die gesättigte Flüssigkeit eine reine Komponente ist, tritt ein Teil der Flüssigkeit durch die Flash-Verdampfung sofort in die Dampfphase über. Aufgrund der Flashverdampfung wird sowohl der erzeugte Dampf als auch der restliche nicht verdampfte Flüssigkeitsteil abgekühlt und ein neues Temperaturniveau einnehmen. Das neue Temperaturniveau wird Sättigungstemperatur genannt und gekennzeichnet das neue thermodynamische Gleichgewicht, das sich nach der Flash-Verdampfung zwischen der zwei neuen Phasen einstellt. Die Abkühlung wegen der Verdampfung ist als Verdampfungskühlung bekannt oder als "Auto-Kühlung" bezeichnet. Wenn die gesättigte Flüssigkeit eine Mehrkomponenten-Flüssigkeit bzw. eine Lösung von leicht flüchtigen und nicht flüchtigen Komponenten ist, werden durch die Flash-Verdampfung nur die leicht flüchtigen Komponenten teilweise getrennt. Nach der Vollendung der Flash-Verdampfung entstehen zwei Phasen. Die eine (Dampfphase) ist reicher an den leicht flüchtigen Komponenten und die andere ist reicher an den nicht flüchtigen Komponenten. Weil die Flash-Verdampfung zur Übersättigung der nicht flüchtigen Komponenten in der Lösung führen kann, besteht es die Möglichkeit, daß die nicht flüchtigen Komponenten auskristallisieren. Zugleich wird die Umwandlung der nicht flüchtigen Komponenten in die kristalline Phase von der Verdampfungskühlung zusätzlich vergünstigt. Gemäß der Zusammensetzung und der Temperatur des Zulaufstroms kann mittels Flash-Verdampfung eine erhebliche Menge des freien TDI verdampft werden.

Der nun teils aufkonzentrierte Zulaufstrom wird auf die temperierte Schüttung des feinkörnigen festen Guts (Rückstand) vorzugsweise als dünner Film verteilt und verdampft. Es sollte erfindungsgemäß vorzugsweise gewährleistet sein, dass die Temperatur der im Prozessraum befindenden Schüttung des feinkörnigen, festen Guts mindestens 5°C über der Siedetemperatur des Zulaufstroms liegt. Auf diese Weise verdampft das TDI und auf dem feinkörnigen, festen Gut verbleibt der feste Rückstand. Die TDI-Brüden fließen vorzugsweise durch einen Brüdendom, der auf dem Wirbelschichtbehälter sitzt. Von dort werden sie einem nachgeschalteten Sprühkondensator zugeführt, wo sie vollständig kondensiert werden. Der Begriff "Brüden", wie er im Zusammenhang mit der vorliegenden Erfindung verwendet wird, umfasst dabei dampfförmiges TDI.

Das heiße feinkörnige, feste Gut, auf dem sich der Rückstand abgelagert hat, wird kontinuierlich einer geeigneten Kühleinheit, wie zum Beispiel einem Dünschichtkühler, einem Scheibenkühler oder einer Mono-Kühl-Schnecke, zugeführt. Die Temperatur des Rückstands befindet sich vor der Kühlung vorzugsweise in einem Bereich von 230 bis 250°C. In der geeigneten Kühleinheit wird der Rückstand bis auf eine Temperatur tiefer als 80°C, vorzugsweise tiefer als 60°C gekühlt. Die geeignete Kühleinheit wird vorzugsweise beim gleichen Druck betrieben, der während dem erfindungsgemäßen Verfahren im Wirbelschichtbehälter vorliegt. Nachgeschaltet zu der geeigneten Kühleinheit wird vorzugsweise ein sogenannter Schleuse-Behälter installiert. Der Schleuse-Behälter hat die Funktion des Ausschleusens des gekühlten Rückstands. Der Betrieb des Schleuse-Behälters ist zeitgesteuert und kann somit kontrolliert werden.

Die vorliegende Erfindung soll nun anhand eines Beispiels erläutert werden, das jedoch nicht einschränkend für den Schutzumfang verstanden werden soll.

Beispiel 1:

Zunächst wird ein Wirbelschichtreaktor bereitgestellt, der die folgenden Maße aufweist: Innendurchmesser 172 mm; Länge 395 mm. Der Wirbelschichtbehälter weist in seinem oberen Bereich zwei Zuführventile auf, durch die der flüssige Rückstand zudosiert werden kann. Die Zuführventile sind als Druckhalteventile ausgebildet. Im Wirbelschichtreaktor befindet sich eine in den Trocknungsraum hineinragende Rotorwelle, an der 10 Rotorblätter mit den folgenden Dimensionen angebracht sind 30x20 (LxB). Um die äußere Wand des Trocknungsbehälters befindet sich eine Heizvorrichtung, die die Wände des Trocknungsbehälters beheizen kann. Ebenso befindet sich in der Rotorwelle eine Heizvorrichtung, die diese beheizen kann. Ebenso am oberen Ende, jedoch an einer anderen Stelle als die Zulaufventile, befindet sich ein sogenannter Brüdendom, der das verdampfte TDI aufnimmt und einem Kondensator zu dessen Gewinnung zuführt. An einem Ende des Wirbelschichtreaktors befindet sich eine Austragsvorrichtung nach der auch ein Schleuse-Behälter installiert ist, sodass der trockene aber heiße Rückstand des feinkörnigen, festen Gutes abgekühlt und ausgeführt werden kann. Das kontinuierliche Zuführen des flüssigen Rückstands der TDI-Herstellung und Gewinnung von TDI im Brüdendom und das Abführen des festen Rückstands ermöglichen einen kontinuierlichen Betrieb des Wirbelschichtreaktors zur Gewinnung von TDI. Das innere Volumen des Trocknungsraums des Wirbelschichtreaktors beträgt 8 Liter.

Vor Beginn des erfindungsgemäßen Verfahrens werden 4 kg eines feinkörnigen, festen Gutes, das aus vorangegangenen Verfahren als Rückstand gewonnen wurde, in den Trocknungsbehälter eingebracht. Die durchschnittliche Partikelgröße des feinkörnigen, festen Rückstands betrug 2,2 mm. Das Volumen des eingebrachten feinkörnigen, festen Gutes betrug hierbei 6,1 Liter.

Anschließend wurde die Temperatur des feinkörnigen, festen Gutes auf 250°C eingestellt und es wurde ein Druck von 133,3 mbar (100 mm Hg) eingestellt. Anschließend wurde durch die Bewegung der Rotorblätter das feinkörnige, feste Gut zu einem Wirbelbett verwirbelt, sodass die Anströmungsgeschwindigkeit an der Spitze der Rotorblätter eine Froude-Zahl von 2,5 ergab.

Der flüssige Rückstand der TDI-Herstellung wurde durch die Zuführventile mit einer Menge von 100 g/min zugeführt. Nachdem 10 Minuten bei der genannten Zulaufgeschwindigkeit der flüssige Rückstand eingesprüht wurde, wurde damit begonnen pro Minute im Mittel 30 g feinkörnigen, festen Guts aus der Apparatur auszutragen. Auf diese Weise ergab sich ein Rückmischverhältnis der Masse des Zulaufstroms der flüssigen Rückstände zur Masse der im Wirbelbettbehälter findenden Schüttung von 1,5.

Die mittlere Verweilzeit der festen Rückstände betrug hierbei etwa 135 Minuten.

Durch das erfindungsgemäße Verfahren konnte TDI mit einem Reinheitsgrad von 99.9 % erhalten werden. Die massenspektrometrische Analyse des abgeführten feinkörnigen, festen Gutes (Rückstand) ergab praktisch keinerlei Rückstände von TDI im festen Rückstand. Selbst nach einem kontinuierlichen Betrieb von 8 Stunden während. 4 Tagen konnten keinerlei Ablagerungen auf den Rotorblättern oder den inneren Wänden des Trocknungsbehälters festgestellt werden. Pro Stunde Betriebszeit konnten im Durchschnitt 4,2 kg TDI gewonnen werden.

**Patentansprüche**

1. Verfahren zur kontinuierlichen thermischen Abtrennung und Gewinnung von Toluylendiisocyanat aus flüssigen Rückständen der Toluylendiisocyanatherstellung in einem durch Rühren einer Schüttung aus feinkörnigem, festem Gut mit einem Rührorgan mechanisch erzeugten Wirbelbett aus dem feinkörnigen, festen Gut in einem Wirbelbettbehälter, **dadurch gekennzeichnet, dass**

   (a) die Umfangsgeschwindigkeit an der Spitze des Rührorgans stets eine Froude-Zahl von größer als 1 ergibt;
   (b) die im Wirbelbettbehälter vorgelegte Schüttung des feinkörnigen, festen Gutes mindestens 20 bis maximal 80 Vol.-% des verfügbaren Volumens des Wirbelbettbehälters belegt;
   (c) das Erhitzen der flüssigen Rückstände auf Verdampfungstemperatur durch direkten Kontakt mit dem vorgeheizten feinkörnigen, festen Gut der im Behälter vorgelegten und gewirbelten Schüttung erfolgt, wobei der Wärmeübertragungskoeffizient mindestens 120 W/m$^2$K beträgt; und
   (d) das Rückmischverhältnis der Masse des Zulaufstroms der flüssigen Rückstände zur Masse der im Wirbelbettbehälter befindenden Schüttung mindestens 0,2 ist.

2. Verfahren nach Anspruch 1, **dadurch gekennzeichnet, dass** das Wirbelbett bei Temperaturen von 150 bis 380°C gehalten wird.

3. Verfahren nach Anspruch 1 oder 2, **dadurch gekennzeichnet, dass** der Druck im Wirbelbettbehälter im Bereich von 25 mbar bis 1000 mbar liegt.

4. Verfahren nach einem der Ansprüche 1 bis 3, **dadurch gekennzeichnet, dass** die mittlere Verweilzeit der Rückstände im Wirbelbett im Bereich von 30 Minuten bis 180 Minuten liegt.

5. Verfahren nach einem der Ansprüche 1 bis 4, **dadurch gekennzeichnet, dass** das mechanisch erzeugte Wirbelbett aus dem feinkörnigen, festen Gut durch ein an einer Rotorwelle angeordnetes Rotorblatt als spezielles Rührorgan erzeugt wird.

6. Verfahren nach Anspruch 5, **dadurch gekennzeichnet, dass** das Wirbelbett durch das spezielle Rührorgan vollständig durchmischt wird und somit permanent in einem feinkörnigen, festen und rieselfähigen Zustand gehalten wird.

7. Verfahren nach einem der Ansprüche 1 bis 6, **dadurch gekennzeichnet, dass** das Wirbelbett ausschließlich aus feinkörnigem, festem Rückstand besteht.

8. Verfahren nach einem der Ansprüche 1 bis 7, **dadurch gekennzeichnet, dass** für die Zufuhr der flüssigen Rückstände in das Wirbelbett eine Einstoffdüsebatterie verwendet wird.

9. Verfahren nach Anspruch 8, **dadurch gekennzeichnet, dass** der Zulaufstrom der Rückstände durch die Einstoffdüsebatterie mit einem Vordruck von grösser 1 bar auf das Wirbelbett eingedüst wird.

10. Verfahren nach einem der Ansprüche 1 bis 8, **dadurch gekennzeichnet, dass** die Energie für die Beheizung der im Wirbelbettbehälter befindenden Schüttung des feinkörnigen, festen Gutes ausschließlich über die beheizte Wand des Wirbelbettbehälters und über das beheizte spezielle Rührorgan eingebracht wird.

**Claims**

1. Method for continuous thermal separation and extraction of toluene diisocyanate from liquid residues of the toluene diisocyanate production in a fluidised bed of fine-grained, solid material in a fluidised bed container, said fluidised bed being generated mechanically by stirring a bulk material made from fine-grained, solid material using a stirrer, **characterised in that**

   a) the peripheral speed at the tip of the stirrer always results in a Froude number of greater than 1;
   b) the bulk material of the fine-grained solid material present in the fluidised bed container occupies at least 20 to a maximum of 80% by volume of the available volume of the fluidised bed container;
   c) the heating of the liquid residues to an evaporation temperature by direct contact with the pre-heated fine-grained, solid material of the swirled bulk material present in the container occurs, wherein the heat transfer coefficient is at least 120 W/m$^2$K; and
   d) the remixing ratio of the mass of the feed flow of the liquid residues to the mass of the bulk material located in the fluidised bed container is at least 0.2.

2. Method according to claim 1, **characterised in that** the fluidised bed is kept at temperatures from 150 to 380°C.

3. Method according to claim 1 or 2, **characterised in that** the pressure in the fluidised bed container ranges from 25 mbar to 1000 mbar.

4. Method according to one of claims 1 to 3, **characterised in that** the average dwell time of the residues in the fluidised bed ranges from 30 minutes to 180 minutes.

5. Method according to one of claims 1 to 4, **characterised in that** the mechanically generated fluidised bed of the fine-grained, solid material is generated by a rotor blade arranged on a rotor shaft as a special stirrer.

6. Method according to claim 5, **characterised in that** the fluidised bed is completely mixed through by the special stirrer and therefore is kept permanently in a fine-grained, solid and pourable state.

7. Method according to any one of claims 1 to 6, **characterised in that** the fluidised bed consists exclusively of fine-grained, solid residue.

8. Method according to any one of claims 1 to 7, **characterised in that** a single-material nozzle battery is used for the supply of the liquid residues into the fluidised bed.

**9.** Method according to claim 8, **characterised in that** the feed flow of the residues is injected onto the fluidised bed through the single-material nozzle battery with a pressure of greater than 1 bar.

**10.** Method according to one of claims 1 to 8, **characterised in that** the energy for the heating of the bulk material of the fine-grained, solid material located in the fluidised bed container is introduced exclusively via the heated wall of the fluidised bed container and via the heated special stirrer.

**Revendications**

**1.** Procédé de séparation thermique en continu et de récupération de diisocyanate de toluylène à partir de résidus liquides de la fabrication de diisocyanate de toluylène dans un lit fluidisé produit mécaniquement par un organe d'agitation par agitation d'une distribution de matériau solide de fine granulométrie à partir du matériau solide de fine granulométrie dans un récipient de lit fluidisé, **caractérisé en ce que** :

(a) la vitesse périphérique à la pointe de l'organe d'agitation donne toujours un nombre de Froude de plus de 1 ;
(b) la distribution présente dans le récipient de lit fluidisé du matériau solide de fine granulométrie occupe au moins 20 jusqu'à au maximum 80 % en volume du volume disponible du récipient de lit fluidisé ;
(c) le chauffage des résidus liquides à la température de vaporisation se fait par contact direct avec le matériau solide de fine granulométrie préchauffé de la distribution présente fluidisée dans le récipient, dans lequel le coefficient de transfert thermique atteint at moins 120 W/m$^2$K; et
(d) le rapport de reconversion de la masse du courant d'alimentation des résidus liquides à la masse de la distribution se trouvant dans le récipient de lit fluidisé est d'au moins 0,2.

**2.** Procédé selon la revendication 1, **caractérisé en ce que** le lit fluidisé est maintenu à des températures de 150 à 380 °C.

**3.** Procédé selon la revendication 1 ou 2, **caractérisé en ce que** la pression dans le récipient de lit fluidisé se situe dans la plage de 25 mbars à 1000 mbars.

**4.** Procédé selon l'une quelconque des revendications 1 à 3, **caractérisé en ce que** le temps de séjour moyen des résidus dans le lit fluidisé se situe dans la plage de 30 minutes à 180 minutes.

**5.** Procédé selon l'une quelconque des revendications 1 à 4, **caractérisé en ce que** le lit fluidisé produit mécaniquement formé du matériau solide de fine granulométrie est généré par une pale de rotor agencée sur un arbre de rotor comme organe d'agitation spécial.

**6.** Procédé selon la revendication 5, **caractérisé en ce que** le lit fluidisé est mélangé intégralement par l'organe d'agitation spécial et est donc maintenu en permanence dans un état de fine granulométrie, solide et coulant.

**7.** Procédé selon l'une quelconque des revendications 1 à 6, **caractérisé en ce que** le lit fluidisé est constitué exclusivement de résidus solides de fine granulométrie.

**8.** Procédé selon l'une quelconque des revendications 1 à 7, **caractérisé en ce que**, pour l'alimentation en résidus liquides dans le lit fluidisé, on utilise une batterie de buses unitaire.

**9.** Procédé selon l'alimentation 8, **caractérisé en ce que** le courant d'alimentation en résidus est introduit par la batterie de buses unitaire avec une pression préalable de plus de 1 bar sur le lit fluidisé.

**10.** Procédé selon l'une quelconque des revendications 1 à 8, **caractérisé en ce que** l'énergie pour le chauffage de la distribution se trouvant dans le récipient de lit fluidisé du matériau solide de fine granulométrie est appliquée exclusivement via la paroi chauffée du récipient de lit fluidisé et via l'organe d'agitation spécial chauffé.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 1014538
- US 2889257 A
- DE 1133721
- DE 1231689
- DE 1243178
- US 3729386 A
- DE 4317669
- US 3457291 A
- DE 2452805
- DE 2915830
- DE 4430951